(19) Europäisches Patentamt
European Patent Office
Office européen des brevets

(11) Veröffentlichungsnummer: **0 183 115**
**B1**

(12) # EUROPÄISCHE PATENTSCHRIFT

(45) Veröffentlichungstag der Patentschrift: **01.06.88**

(51) Int. Cl.⁴: **C 07 C 119/048**, C 07 C 118/02, C 08 G 18/77

(21) Anmeldenummer: **85114308.1**

(22) Anmeldetag: **11.11.85**

(54) **Gegebenenfalls in 3-Stellung Methyl-substituierte Bis-(4-isocyanatophenoxy)-alkane, ein Verfahren zu ihrer Herstellung und ihre Verwendung bei der Herstellung von Polyurethankunststoffen.**

(30) Priorität: **23.11.84 DE 3442689**
**18.07.85 DE 3525606**

(43) Veröffentlichungstag der Anmeldung:
**04.06.86 Patentblatt 86/23**

(45) Bekanntmachung des Hinweises auf die Patenterteilung:
**01.06.88 Patentblatt 88/22**

(84) Benannte Vertragsstaaten:
**BE DE FR GB IT NL SE**

(56) Entgegenhaltungen:
**DE - A - 3 138 421**
**US - A - 3 449 395**

**JOURNAL OF THE AMERICAN CHEMICAL SOCIETY, Band 74, Nr. 20, 23. Oktober 1952, Seiten 5229-5230; F.H. McMILLAN: "Diaryloxyalkane derivatives. Some miscellaneous diphenoxypropanes"**
**J. LIEBIGS: "Annalen der Chemie", Band 562, 1949, Seiten 75-136, Verlag Chemie GmbH, Weinheim, DE; W. SIEFKEN: "Mono- und Polyisocyanate"**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **König, Klaus, Dr., Heymannstrasse 50, D-5090 Leverkusen 1 (DE)**
Erfinder: **Heitkämper, Peter, Dr., Fliederweg 14, D-4047 Dormagen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ausgewählte, gegebenenfalls in 3-Stellung Methyl-substituierte Bis-(4-isocyanatophenoxy)-alkane, ein Verfahren zu ihrer Herstellung durch Umsetzung von gegebenenfalls in 3-Stellung Methyl-substituierten Bis-(4-aminophenoxy)-alkanen oder ihrer Addukte mit Chlorwasserstoff oder Kohlendioxid mit Phosgen, sowie ihre Verwendung als Aufbaukomponente bei der Herstellung von Polyurethankunststoffen.

Die Eigenschaften von Polyurethankunststoffen, insbesondere von Polyurethanelastomeren hängen u.a. wesentlich von der Natur des bei der Herstellung der Kunststoffe eingesetzten Polyisocyanats ab. Besonders hochwertige Polyurethanelastomere werden so beispielsweise bei Verwendung von 1,5-Diisocyanatonaphthalin als Diisocyanatkomponente erhalten. Insbesondere zeichnen sich Giesselastomere auf Basis dieses Diisocyanats durch hervorragende mechanische Eigenschaften aus [vgl. z.B. Becker, Braun, Kunststoff-Handbuch, Band 7, 2. Auflage, (1983), Carl Hanser Verlag].

1,5-Diisocyanatonaphthalin ist jedoch mit dem Nachteil behaftet, dass der zu seiner Herstellung eingesetzte Grundrohstoff, Naphthalin, nur in beschränkten Mengen zur Verfügung steht. Die Nitrierung von Naphthalin führt ausserdem zwangsläufig zu einem Isomerengemisch von Nitronaphthalinen, aus dem 1,5-Dinitronaphthalin isoliert werden muss. Die destillative Reinigung des 1,5-Diisocyanatonaphthalins, welches aus der Dinitroverbindung durch Hydrierung und anschliessende Phosgenierung des resultierenden Diamins erhalten wird, bereitet ebenfalls Schwierigkeiten, da es zur Sublimation neigt. Dies alles führt dazu, dass 1,5-Diisocyanatonaphthalin mit hohen Kosten belastet ist.

Auch die Verarbeitung von 1,5-Diisocyanatonaphthalin ist häufig problematisch, weil sein Schmelzpunkt und sein Dampfdruck relativ hoch sind. Diese Eigenschaften erlauben es oft nicht, 1,5-Diisocyanatonaphthalin ohne weiteres als Schmelze umzusetzen. Es sind dann technisch aufwendige Verarbeitungsmethoden und Schutzmassnahmen erforderlich, um chemische und arbeitsmedizinische Schwierigkeiten zu vermeiden.

Es hat daher nicht an Versuchen gefehlt, für 1,5-Diisocyanatonaphthalin als Diisocyanatkomponente bei der Herstellung hochwertiger Polyurethankunststoffe einen gleichwertigen Ersatz zu finden.

So ist beispielsweise in DE-OS 3 138 421 und DE-OS 3 138 422 die Herstellung von Polyurethan-Elastomeren unter Verwendung von 4,4'-Diisocyanatodiphenylethan-(1,2) als Diisocyanat-Komponente beschrieben. Zwar können mit diesem Diisocyanat Kunststoffe mit guten mechanischen Eigenschaften erhalten werden; jedoch ist die Herstellung von 4,4'-Diisocyanato-diphenylmethan-(1,2) sehr umständlich und aufwendig und bis jetzt auch technisch schwer realisierbar.

Weiterhin sind zahlreiche Versuche unternommen worden, das vergleichsweise kostengünstige 4,4'-Diisocyanato-diphenylmethan anstelle von 1,5-Diisocyanatonaphthalin zur Herstellung von hochwertigen Polyurethanelastomeren zu verwenden, jedoch sind bislang alle Versuche gescheitert, auf Basis dieses Diisocyanats Polyurethanelastomere herzustellen, die bezüglich ihrer mechanischen und thermischen Eigenschaften den Polyurethanelastomeren auf Basis von 1,5-Diisocyanatonaphthalin entsprechen.

Es war daher die Aufgabe der vorliegenden Erfindung, neue Diisocyanate zur Verfügung zu stellen, welche bezüglich ihrer Eignung zur Herstellung von hochwertigen Polyurethanelastomeren dem 1,5-Diisocyanatonaphthalin vergleichbar sind, und welche sich nach einfachen Verfahren kostengünstig herstellen lassen.

Diese Aufgabe konnte durch die Bereitstellung von ausgewählten, gegebenenfalls in 3-Stellung Methyl-substituierten Bis-(4-isocyanatophenoxy)-alkanen bzw. dem Verfahren zu ihrer Herstellung gelöst werden.

Gegenstand der Erfindung sind gegebenenfalls in 3-Stellung Methyl-substituierte Bis-(4-isocyanatophenoxy)-alkane der allgemeinen Formel

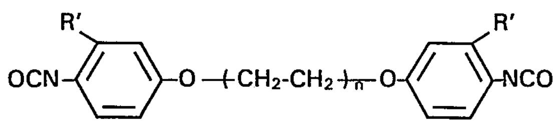

in der

R' für Wasserstoff oder eine Methylgruppe steht, wobei beide R' jeweils die gleiche Bedeutung haben, und

n für 1, 2 oder 3 steht.

Gegenstand der Erfindung ist auch ein Verfahren zur Herstellung von Bis-(4-isocyanatophenoxy)-alkanen der genannten Formel, dadurch gekennzeichnet, dass man Bis-(4-aminophenoxy)-alkane der allgemeinen Formel

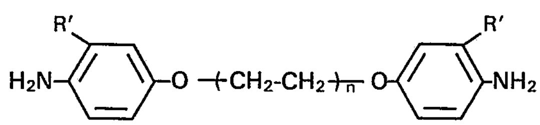

in der R' und n die obengenannte Bedeutung haben, oder deren Addukte mit Chlorwasserstoff oder Kohlendioxid in an sich bekannter Weise mit Phosgen umsetzt.

Gegenstand der Erfindung ist auch die Verwendung von gegebenenfalls in 3-Stellung Methyl-substituierten Bis-(4-isocyanatophenoxy)-alkanen der genannten Formel als Aufbaukomponente bei der Herstellung von Polyurethankunststoffen nach dem Isocyanat-Polyadditionsverfahren.

Aus J. Am. Chem. Soc. 74 (1952), 5229-5320 ist zwar 1,3-Bis-(4-isocyanatophenoxy)-propan bereits bekannt, jedoch unterscheidet sich dieses Diisocyanat bezüglich seiner Eignung zur Herstellung von hochwertigen Polyurethanelastomeren äussert nachteilhaft von den erfindungsgemässen Diisocyanaten. Überraschenderweise hängen die mechanischen Eigenschaften der aus den Diisocyanaten hergestellten Polyurethanelastomere entscheident von der Frage ab, ob es sich bei der Oligomethylenbrücke zwischen den beiden Isocyanato-phenoxy-Resten

um eine solche mit einer geraden oder einer ungeraden Anzahl an Kohlenstoffatomen handelt. Nur im Falle des Vorliegens von Oligomethylen-Resten mit einer geraden Anzahl an Kohlenstoffatomen liegen Diisocyanate vor, die die Herstellung von hochwertigen Elastomeren gestatten.

Die bei der Durchführung des erfindungsgemässen Verfahrens als Ausgangsmaterial einzusetzenden Diamine ohne Methyl-Substituenten sind bekannt und in der Literatur beschrieben, beispielsweise im Journal für Praktische Chemie, Band 27, (1883), Seiten 206 und 207, im Archiv der Pharmazie, Band 236 (1898), Seiten 260 bis 262 oder in Ophthalmologica, Band 136 (1958), Seiten 332 bis 344. Die Diamine werden üblicherweise aus den entsprechenden Dinitroverbindungen durch Reduktion mit unedlen Metallen, z.B. Zinn oder Eisen, in Gegenwart von Säuren oder durch katalytische Hydrierung hergestellt. Selbstverständlich können für das erfindungsgemässe Verfahren die Diamine auch nach jeder beliebigen anderen Methode hergestellt werden.

Die den, keine Methyl-Substituenten aufweisenden, Diaminen zugrundeliegenden Dinitroverbindungen, sind leicht zugänglich. Sie können beispielsweise durch Kondensation von Alkali-4-nitrophenolat mit Alkan-dihalogeniden, durch Umsetzung von 4-Nitrochlorbenzol mit Alkandiolen in Gegenwart von Basen oder durch Umsetzung von 4-Nitrochlorbenzol mit (4-Nitrophenoxy)-alkanolen in Gegenwart von Basen hergestellt werden. Weitere Herstellungsverfahren sind in der Literatur beschrieben, z.B. in «The chemistry of the ether linkage» (Herausgeber S. Patai, Interscience Publishers, 1967), Seiten 445 bis 498.

Die bei der Durchführung des erfindungsgemässen Verfahrens als Ausgangsmaterial einzusetzenden, in 3-Stellung Methyl-Substituenten aufweisenden Diamine sind zum Teil bekannt und in der Literatur beschrieben, 1,4-Bis-(4-amino-3-methyl-phenoxy)-butan beispielsweise in Brit. J. Pharmacol. 11, 375 bis 378 (1956).

Auch diese Diamine werden üblicherweise aus den entsprechenden Dinitroverbindungen durch Reduktion mit unedlen Metallen, z.B. Zinn oder Eisen, in Gegenwart von Säuren oder durch katalytische Hydrierung hergestellt. Selbstverständlich können auch diese Diamine auch nach jeder beliebigen anderen Methode hergestellt werden.

Die den Methyl-substituierten Diaminen zugrundeliegenden Dinitroverbindungen, sind leicht zugänglich. Sie können beispielsweise durch Kondensation von Alkali-4-nitro-3-methylphenolat mit Alkan-dihalogeniden, durch Umsetzung von 4-Nitro-3-methyl-chlorbenzol mit Alkandiolen in Gegenwart von Basen oder durch Umsetzung von 4-Nitro-3-methyl-chlorbenzol mit (4-Nitro-3-methyl-phenoxy)-alkanolen in Gegenwart von Basen hergestellt werden. Weitere Herstellungsverfahren sind in der Literatur beschrieben, z.B. in «The Chemistry of the ether linkage» (Herausgeber S. Patai, Interscience Publishers 1967), Seiten 555 bis 498.

Beim erfindungsgemässen Verfahren werden Diamine der allgemeinen Formel

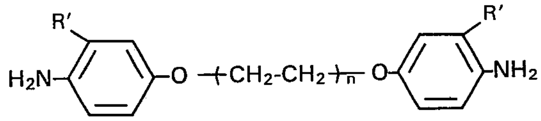

eingesetzt,

für welche n für 1, 2 oder 3, vorzugsweise für 1 oder 2 und besonders bevorzugt für 1 steht, und für welche R' die bereits obengenannte Bedeutung hat, eingesetzt. Die bei Verwendung dieser Diamine erhaltenen, erfindungsgemässen Diisocyanate entsprechen der allgemeinen Formel

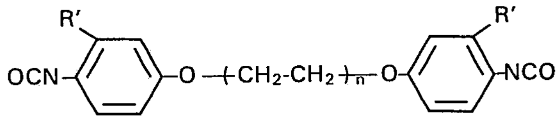

in welcher

n die soeben genannte Bedeutung bzw. bevorzugte Bedeutung hat, und R' den obengemachten Angaben entspricht.

Typische Beispiele für beim erfindungsgemässen Verfahren einzusetzende Ausgangsamine sind 1,2-Bis-(4-aminophenoxy)-ethan, 1,4-Bis-(4-aminophenoxy)-butan oder 1,6-Bis-(4-aminophenoxy)-hexan. Beispiele für Methyl-substituierte Diamine sind 1,2-Bis-(4-amino-3-methyl-phenoxy)-ethan oder 1,4-Bis-(4-amino-3-methyl-phenoxy)-butan. 1,2-Bis-(4-aminophenoxy)-ethan und 1,2-Bis-(4-amino-3-methyl-phenoxy)-ethan sind die bevorzugten beim erfindungsgemässen Verfahren einzusetzenden Diamine. Dementsprechend handelt es sich bei 1,2-Bis-(4-isocyanatophenoxy)-ethan und 1,2-Bis-(4-isocyanato-3-methyl-phenoxy)-ethan um die besonders bevorzugten erfindungsgemässen Diisocyanate.

Die erfindungsgemäss zu phosgenierenden Diamine können beim erfindungsgemässen Verfahren in technischer Reinheit, wie sie bei ihrer Herstellung anfallen, oder auch in gereinigter Form zum Einsatz gelangen. Die Reinigung kann beispielsweise durch Lösen in Dimethylformamid und anschliessendes Ausfällen mit Wasser oder destillativ erfolgen.

Beim erfindungsgemässen Verfahren können die Diamine als solche oder auch in Form ihrer Additionsverbindungen mit Chlorwasserstoff oder mit Kohlendioxid zum Einsatz gelangen. Die erfindungsgemässe Phosgenierung erfolgt im übrigen nach an sich bekannten Methoden, wie sie beispielsweise in Liebigs Annalen der Chemie, Band 562, Jahrgang 1949, Seiten 75 bis 109, in Ullmanns Encyclopädie der technischen Chemie, Band 14, 4. Auflage, 1977, Seiten 350 bis 354 oder in Houben-Weyl, Methoden der organischen Chemie, Band E4, 4. Auflage, 1983, Seiten 741 bis 753 beschrieben sind.

Die Umsetzung kann kontinuierlich oder diskontinuierlich bevorzugt in Gegenwart eines inerten Lösungsmittels durchgeführt werden. Geeignete Lösungsmittel sind die für Phosgenierungen üblicherweise verwendeten Lösungsmittel, wie aliphatische, cycloaliphatische oder aromatische Kohlenwasserstoffe, Halogenkohlenwasserstoffe, Nitrokohlenwasserstoffe, aliphatisch-aromatische Ether, aromatische Ether, Carbonsäureester, Carbonsäurenitrile, Sulfone, Phosphorsäurehalogenide oder Phosphorsäureester. Als Beispiele für geeignete Lösungsmittel seien genannt: Trimethylpentan, Decahydronaphthalin, Toluol, 1,2-Dichlorethan, Chlorbenzol, Chlortoluol, 1,2-Dichlorbenzol, Nitrobenzol, Anisol, Phenetol, Diphenylether, Essigsäurebutylester, Tetramethylensulfon, Phosphoroxychlorid, Phosphorsäuretrimethylester. Bevorzugt wird technisches Chlorbenzol oder technisches 1,2-Dichlorbenzol als Lösungsmittel verwendet. Beliebige Gemische der beispielhaft genannten Lösungsmittel können selbstverständlich ebenfalls eingesetzt werden. In den meisten der beispielhaft genannten Lösungsmittel weisen die Bis-(4-aminophenoxy)-alkane bei niedrigen Temperaturen nur eine geringe, zum Teil eine äusserst beschränkte Löslichkeit auf. Bei der Durchführung des erfindungsgemässen Verfahrens erfüllen demzufolge die beispielhaft genannten Lösungsmittel insbesondere bei den niedrigen Phosgeniertemperaturen vor allem die Funktion eines Suspendiermittels für das Diamin bzw. dessen Chlorwasserstoff- oder Kohlendioxid-Addukte und erst bei höheren Temperaturen und mit zunehmender Überführung des Ausgangsmaterials in das Diisocyanat die Funktion eines echten Lösungsmittels für das Ausgangsmaterial und insbesondere das Verfahrensprodukt.

Bei den beim erfindungsgemässen Verfahren einzusetzenden Gemischen aus zu phosgenierendem Ausgangsmaterial und Lösungsmittel handelt es sich im allgemeinen um «Lösungssuspensionen» mit einem Gehalt an Diamin bzw. Diaminaddukt der oben beispielhaft genannten Art von ca. 2 bis 70 Gew.-%. Der Begriff «Lösungssuspension» soll andeuten, dass die Ausgangsmaterialien, insbesondere im Falle der bevorzugten Verwendung der Diamine, teilweise gelöst und teilweise suspendiert vorliegen.

Die erfindungsgemässe Phosgenierungsreaktion kann beispielsweise nach dem bekannten Prinzip der «Kalt-Heiss-Phosgenierung» zweistufig oder nach dem Prinzip der «Heiss-Phosgenierung» einstufig erfolgen. Bei der «Kalt-Heiss-Phosgenierung» erfolgt die Umsetzung des zu phosgenierenden Ausgangsmaterials zu Beginn der Reaktion im allgemeinen bei −20 bis +40°C, vorzugsweise −10 bis +30°C und die anschliessende Heissphosgenierung bei 40 bis 260°C, vorzugswseise 80 bis 220°C. Bei dieser «Kalt-Heiss-Phosgenierung» kann der Bereich zwischen der Anfangstemperatur und der erhöhten Temperatur gleichmässig oder in Sprüngen durchlaufen werden.

Bei der «Heissphosgenierung» kommt das zu phosgenierende Ausgangsmaterial mit dem Phosgen sofort bei Temperaturen von 40 bis 260°C, vorzugsweise 80 bis 220°C in Kontakt.

Die genannte «Kalt-Heiss-Phosgenierung» ist die bevorzugte Verfahrensweise im Falle der Phosgenierung des Diamins (anstelle der Chlorwasserstoff- bzw. Kohlendioxid-Addukte). Hierbei erfolgt bei den genannten tiefen Temperaturen keine nennenswerte Umsetzung zwischen dem suspendierten Diamin und dem zugefügten Phosgen. Erst bei der nachfolgenden Erhöhung der Temperatur beginnt das Diamin mit dem Phosgen entsprechend seiner zunehmenden Löslichkeit zu reagieren.

Bei allen Varianten der erfindungsgemässen Phosgenierung erfolgt diese vorzugsweise unter Normaldruck oder bei erhöhtem Druck. Der Reaktionsdruck liegt im allgemeinen bei 0,9 bis 100, vorzugsweise 1 bis 60 bar.

Im allgemeinen wird bei der erfindungsgemässen Phosgenierungsreaktion das zu phosgenierende Ausgangsmaterial mit einer 1- bis 10fachen, vorzugsweise 1,05- bis 6fachen stöchiometrischen Menge an Phosgen zusammengebracht. Die genannte Phosgenmenge kann in einer Portion oder auch in Teilmengen in das Reaktionsgemisch eingegeben werden. Es kann vorteilhaft sein, z.B. bei einer diskontinuierlichen Arbeitsweise, erst einen Teil der zu verwendenden Phosgenmenge in das Reaktionsgemisch einzubringen und den restlichen Anteil in weiteren Portionen oder stetig über einen längeren Zeitraum verteilt in das Reaktionsgemisch einzubringen.

Grundsätzlich lässt sich die erfindungsgemässe Phosgenierung der Diamine durch Zugabe von Katalysatoren, beispielsweise Dimethylformamid, und/oder Säureakzeptoren, beispielsweise Pyridin, beschleunigen. Im allgemeinen jedoch sind die Reaktionsgeschwindigkeiten bei der Phosgenierung des Diamins auch ohne Zugabe eines derartigen Katalysators ausreichend.

Die Reaktionsdauer bei der erfindungsgemässen Phosgenierung ist von den angewandten Reaktionsbedingungen, insbesondere von den Reaktionstemperaturen, vom Phosgen-Überschuss, von der Verdünnung mit Lösungsmittel und von gegebenenfalls zugegerbenen Katalysatoren und/oder Säureakzeptoren abhängig.

Nach Beendigung der Phosgenierungsreaktion wird das Reaktionsgemisch in an sich bekannter Weise durch Abtrennung von gasförmigen Bestandteilen (Chlorwasserstoff, überschüssiges Phosgen) und destillativer Entfernung des Lösungsmittels aufgearbeitet. Vor der destillativen Entfernung des Lösungsmittels können gegebenenfalls vorliegende feste Nebenprodukte durch Filtration oder Zentrifugieren entfernt werden. Das nach der destillativen Entfernung des Lösungsmittels als Destillationsrückstand anfallende Rohprodukt kann gewünschtenfalls durch Umkristallisieren aus einem geeigneten inerten Lösungsmittel, beispielsweise Toluol oder vorzugsweise destillativ gereinigt werden. So lässt sich 1,2-Bis-(4-isocyanatophenoxy)-ethan beispielsweise bei einem Druck von 3 bis 5 mbar bei Temperaturen von 240 bis 250°C als farbloses Destillat gewinnen, welches alsbald zu einer Festsubstanz mit einem Schmelzpunkt von ca. 98 bis 99°C erstarrt.

Obwohl es sich bei den erfindungsgemässen Diisocyanaten um thermostabile Substanzen handelt, kann es zweckmässig sein, die Reindestillation der Diisocyanate ohne grosse Temperaturbelastung, beispielsweise mittels eines Dünnschichtverdampfers durchzuführen. Gewünschtenfalls können die erfindungsgemässen Diisocyanate nach ihrer Reindarstellung auch durch Tempern bei Temperaturen von 160 bis 250°C, vorzugsweise 180 bis 230°C von störenden Nebenprodukten, beispielsweise thermolabilen, chlorhaltigen Verbindungen befreit werden.

Bei der erfindungsgemässen Verwendung der neuen Diisocyanate zur Herstellung von Polyurethankunststoffen, insbesondere von massiven oder zellförmigen Polyurethanelastomeren werden die erfindungsgemässen Diisocyanate anstelle der bislang für diesen Verwendungszweck eingesetzten Diisocyanate mit den bekannten Reaktionspartnern zur Umsetzung gebracht [vgl. diesbezüglich beispielsweise die bereits eingangs genannten Literaturstellen oder auch «Kunststoff-Handbuch», Band VII, «Polyurethane» von Vieweg und Höchtlen, Carl Hanser Verlag München (1966), insbesondere Seiten 206 bis 297].

So erfolgt beispielsweise die Herstellung von Polyurethanelastomeren unter Verwendung des erfindungsgemässen Diisocyanats durch dessen Umsetzung mit

a) di- oder trifunktionellen Polyhydroxylverbindungen des Molekulargewichtsbereichs 400 bis 10 000, vorzugsweise 800 bis 3000, vorzugsweise den entsprechenden Polyhydroxypolyestern oder Polyhydroxypolyethern,

b) Kettenverlängerungsmitteln des Molekulargewichtsbereichs 60 bis 399, d.h. mit im Sinne der Isocyanat-Additionsreaktion difunktionellen Verbindungen mit alkoholischen Hydroxylgruppen oder primären bzw. sekundären Aminogruppen, gegebenenfalls in Gegenwart von

c) weiteren aus der Chemie der Polyurethanelastomeren an sich bekannten Hilfs- und Zusatzmitteln.

Die Umsetzung kann nach dem bekannten Prepolymerverfahren [Umsetzung des Diisocyanats mit der Komponente a)] unter Einhaltung eines Äquivalentverhältnisses von Isocyanatgruppen zu gegenüber Isocyanatgruppen reaktionsfähigen Gruppen von grösser als 1,3:1 und anschliessende Umsetzung des so erhaltenen NCO-Prepolymeren mit der Komponente b) oder aber auch einstufig durch Umsetzung des Diisocyanats mit einem Gemisch der Komponenten a) und b) erfolgen. Bei beiden Varianten liegt das Äquivalentverhältnis von Isocyanatgruppen zur Gesamtmenge der gegenüber Isocyanatgruppen reaktionsfähigen Gruppen im allgemeinen bei 0,8:1 bis 1,3:1, vorzugsweise 0,95:1 bis 1,1:1. Die Temperaturen, bei welchen diese Umsetzungen durchgeführt werden, liegen im allgemeinen bei 60 bis 180°C, vorzugsweise bei 80 bis 150°C. Die Umsetzungen können in Anwesenheit oder auch in Abwesenheit von geeigneten inerten Lösungsmitteln erfolgen.

Bei den mit den erfindungsgemässen Diisocyanaten hergestellten Polyurethankunststoffen, insbesondere Polyurethanelastomeren kann es sich sowohl um massive als auch um zellförmige Produkte handeln. Die Herstellung beider Typen von Polyurethanelastomeren erfolgt nach den bekannten Verfahren, wie sie beispielsweise in der zuletzt genannten Literaturstelle beschrieben sind. So erfolgt beispielsweise die Herstellung von zellförmigen Polyurethanelastomeren unter Verwendung bzw. Mitverwendung von Wasser als Kettenverlängerungsmittel.

Die mit den erfindungsgemässen Diisocyanaten hergestellten Kunststoffe, insbesondere Elastomeren, die mit solchen erfindungsgemässen Diisocyanaten hergestellt sind, bei denen der genannte Kohlenwasserstoffrest R eine Ethylen-, eine Tetramethylen- oder eine Hexamathylen-Gruppe darstellt, besitzen hochwertige mechanische und thermische Eigenschaften. Sind sind deshalb hervorragend geeignet für Feder- und Dämpfungselemente, Puffer, Radbeläge, Dichtungen, Schuhsohlen und ähnliche Einsatzgebiete, bei denen das Material extremen mechanischen und thermischen Beanspruchungen ausgesetzt ist.

Die folgenden Beispiele sollen die Erfindung näher erläutern. Prozentangaben beziehen sich auf Gewichtsprozente.

*Beispiel 1*

[Herstellung von 1,2-Bis-(4-isocyanato-phenoxy)-ethan]

In einer 6-l-Laborphosgenierapparatur wurden 366 g kristallines 1,2-Bis-(4-aminophenoxy)-ethan (analytisch bestimmte Reinheit: 99,8%) vorgelegt und mit 4,4 l wasserfreiem Chlorbenzol versetzt. Die Suspension wurde in einem Eisbad abgekühlt. Bei 0 bis 10°C wurden in die Suspension zügig 320 g Phosgen eingeleitet. Dann wurde das Gemisch unter Rühren und langsamem Einleiten von Phosgen (40 bis 50 g/h) im Verlauf von 2,5 Stunden zum Rückfluss erhitzt. Nach weiteren 1,5 Stunden Phosgenieren (40 bis 50 g Phosgen/h) am Rückfluss war eine klare Lösung entstanden. Das fertige Reaktionsgemisch wurde destillativ von überschüssigem Phosgen und vom grössten Teil des Lösungsmittels befreit und dann auf 50°C abgekühlt, wobei Kristalle ausfielen. Aus der breiigen Suspension wurde das restliche Chlorbenzol im Vakuum bei 50°C vollständig abdestilliert. Dabei wurden als Rückstand 446 g Rohprodukt in Form von schwach bräunlichen Kristallen vom Schmelzpunkt 96 bis 97°C erhalten. Das Rohprodukt wies einen Gehalt an hydrolysierbarem Chlor von 0,13% auf.

Von dem so gewonnenen rohen Diisocyanat wurden 420 g in eine Vakuumdestillation eingesetzt. Dabei destillierte 1,2-Bis-(4-isocyanatophenoxy)-ethan bei 1 mbar und 210 bis 225°C ohne Vorlauf als farblose Flüssigkeit über, die rasch zu Kristallen vom Schmelzpunkt 98 bis 99°C erstarrte.

Ausbeute: 411 g (98,5% der Theorie)
$C_{16}H_{12}N_2O_4$ (296,3)
hydr. Chlor: 170 ppm
NCO-Gehalt: ber.: 28,35% gef.: 28,2%
28,3%

Das Diisocyanat liess sich praktisch rückstandsfrei redestillieren. Das Destillat besass einen Gehalt an hydrolisierbarem Chlor von 50 ppm.

Als Rückstand der ersten Destillation blieben 6 g dunkel gefärbtes Festprodukt vom Schmelzpunkt 350 bis 360°C (Zers.) zurück.

*Beispiel 2*

[Herstellung von 1,2-Bis-(4-isocyanato-phenoxy)-ethan]

Es wurde ungereinigtes 1,2-Bis-(4-Aminophenoxy)-ethan verwendet, das durch katalytische Hydrierung von 1,2-Bis-(4-nitrophenoxy)-ethan erhalten wurde. Das Diamin besass eine analytisch bestimmte Reinheit von 95,4 Gew.-%. In einer 6-l-Laborphosgenierapparatur wurden 366 g Diamin vorgelegt und mit 4,4 l wasserfreiem o-Dichlorbenzol versetzt. Die Suspension wurde abgekühlt und bei 0 bis 10°C zügig mit 310 g Phosgen versetzt. Dann wurde das Gemisch unter Rühren und langsamem Einleiten von Phosgen (40 bis 50 g/h) im Verlauf von 3,5 Stunden zum Rückfluss erhitzt und am Rückfluss eine weitere Stunde phosgeniert. Die fertige, dunkel gefärbte Reaktionslösung enthielt einen unlöslichen, flockigen Niederschlag. Aus dem Gemisch wurden überschüssiges Phosgen und 2 l Dichlorbenzol abdestilliert. Nach dem Abkühlen auf Raumtemperatur wurde der Niederschlag abgesaugt, mit Dichlorbenzol gewaschen und im Vakuum getrocknet: 19,7 g dunkles, amorphes Festprodukt.

Die klare Reaktionslösung wurde im Vakuum destilliert. Nach einem Vorlauf von Dichlorbenzol destillierten 410 g 1,2-Bis-(4-isocyanatophenoxy)-ethan (96,8% der Theorie) als schwach gelbliche Flüssigkeit, die rasch erstarrte.

Schmelzpunkt: 95 bis 97°C
hydr. chlor: 710 ppm
NCO-Gehalt: 28,4%
Destillationsrückstand: 8 g dunkles Festprodukt

Das Diisocyanat wurde redestilliert und ergab fast farblose Kristalle vom Schmelzpunkt 98 bis 99°C mit einem Gehalt an hydrolysierbarem Chlor von 60 ppm.

*Beispiel 3*

[Herstellung von 1,4-Bis-(4-isocyanato--phenoxy)-butan]

Es wurden 408 g 1,4-Bis-(4-aminophenoxy)-butan (analytisch bestimmte Reinheit = 99,9%) entsprechend Beispiel 1 mit Phosgen umgesetzt. Dabei wurden 490 g Rohprodukt in form bräunlicher Kristalle vom Schmelzpunkt 95 bis 97°C erhalten. Das Rohprodukt wies einen Gehalt an hydrolysierbarem Chlor von 0,22% auf.

Von dem so gewonnenen rohen Diisocyanat wurden 450 g in eine Vakuumdestillation eingesetzt. Dabei destillierte 1,4-Bis-(4-isocyanatophenoxy)-butan bei 1 mbar und 220 bis 230°C ohne Vorlauf als farblose Flüssigkeit über, die rasch zu Krisitallen vom Schmelzpunkt 97 bis 98°C erstarrte.

Ausbeute: 436 g (97,7% der Theorie)
$C_{18}H_{16}N_2O_4$ (3,24,3)
hydr. Chlor: 190 ppm
NCO-Gehalt: ber.: 25,9% gef.: 26,0%
25,9%

Das Diisocyanat liess sich praktisch rückstandsfrei redestillieren. Das Destillat besass einen Gehalt an hydrolysierbarem Chlor vom 70 ppm.

*Beispiel 4*

[Herstellung von 1,6-Bis-(4-isocyanato--phenoxy)-hexan]

Es wurden 450 g 1,6-Bis-(4-aminophenoxy)-hexan (analytisch bestimmte Reinheit: 96,1%) entsprechend Beispiel 1 mit Phosgen umgesetzt. Dabei wurden 519 g Rohprodukt in Form bräunlicher Kristalle vom Schmelzpunkt 86 bis 88°C erhalten. Das Rohprodukt wies einen Gehalt an hydrolysierbarem Chlor von 0,27% auf.

Von dem so gewonnenen rohen Diisocyanat wurden 480 g in eine Vakuumdestillation eingesetzt. Dabei destillierte 1,6-Bis-(4-isocyanatophenoxy)-hexan bei 0,3 mbar und 238 bis 244°C ohne Vorlauf als fast farblose Flüssigkeit über, die rasch zu Kristallen vom Schmelzpunkt 88 bis 89°C erstarrte.

Ausbeute: 499 g (95,7% der Theorie)
$C_{20}H_{20}N_2O_4$ (352,4)
hydr. Chlor: 140 ppm
NCO-Gehalt: ber.: 23,8% gef.: 23,8%
23,7%

Das Diisocyanat liess sich praktisch rückstandsfrei redestillieren. Das Destillat war farblos und besass einen Gehalt an hydrolysierbarem Chlor von 60 ppm.

Beispiel 5

a) [Herstellung von 1,2-Bis-(4-amino-3-methylphenoxy)-ethan]

In einer Destillationsapparatur mit Wasserabscheider wurde ein Gemisch von 1989 g 4-Nitro-3-methyl-phenol, 2,5 l Ethylenglykol, 0,5 l Toluol und 1222 g 1,2-Dibromethan vorgelegt und unter Rühren am Rückfluss (ca. 130°C) azeotrop entwässert. Dann wurde das am Rückfluss siedende Gemisch im Verlauf von 2,5 Stunden unter Wasserabtrennung mit 1040 g 50%iger wässriger Natronlauge versetzt. Anschliessend wurde das Reaktionsgemisch unter Zugabe von weiteren 244 g 1,4-Dibromethan und 104 g 50%iger wässriger Natronlauge noch 4,5 Stunden bei 130°C gerührt, dann auf Raumtemperatur abgekühlt und unter kräftigem Rühren mit 3 l Wasser versetzt. Die ausgefallenen Kristalle wurden abfiltriert, mit Wasser und Methanol gewaschen und getrocknet: 1880 g (87% der Theorie) 1,2-Bis-(4-nitro-3-methylphenoxy)-ethan in Form von sandfarbenen Kristallen vom Schmelzpunkt 182°C.

$C_{18}H_{16}N_2O_6$ (332,3)

| | | | | |
|---|---|---|---|---|
| ber.: | C 57,83 | H 4,85 | N 8,43 | O 28,89 |
| gef.: | C 57,8 | H 4,8 | N 8,4 | O 28,6 |

In einem Hydrierautoklaven wurde eine Lösung von 1695 g der Dinitroverbindung in 5 l Dimethylformamid vorgelegt, mit Raney-Nickel versetzt und unter Rühren durch Aufdrücken von Wasserstoff umgesetzt. Die Hydrierung wurde bei 40 bis 50 bar 2 Stunden bei 75°C und dann 1 Stunde bei 90°C durchgeführt. Anschliessend wurde nach Entspannen des Autoklaven das heisse Reaktionsgemisch entommen, durch Filtrieren vom Katalysator befreit und auf Raumtemperatur abgekühlt. Dabei fielen Kristalle aus, die abgesaugt und im Vakuum getrocknet wurden. Die Mutterlauge wurde im Vakuum eingedampft, wobei zusätzliches Produkt zurückblieb. Insgesamt wurden 1365 g 1,2-Bis-(4-amino-3-methyl-phenoxy)-ethan in Form von bräunlichen Kristallen erhalten. Durch Titration mit Perchlorsäure in Eisessig, sowie chromatographisch wurde der Reinheitsgrad des Produktes mit 98,6% festgestellt (Ausbeute: 97% der Theorie, bezogen auf eingesetzte Dinitroverbindungen). Durch destillative Reinigung des Diamins (Siedepunkt; 195 bis 200°C bei 0,2 mbar) wurden praktisch farblose Kristalle vom Schmelzpunkt 106 bis 107°C erhalten.

$C_{16}H_{20}N_2O_2$ (272,3)

b) [Herstellung von 1,2-Bis-(4-isocyanato-3-methyl-phenoxy)-ethan]

In einer 6-l-Laborphosgenierapparatur wurde eine Lösung von 350 g Phosgen in 3,8 l wasserfreiem Chlorbenzol vorgelegt und unter Kühlung bei 0 bis 10°C mit 408 g 1,2-Bis-(4-amino-3-methyl-phenoxy)-ethan (analytisch bestimmte Reinheit: 98,6%) versetzt. Unter Einleiten von zusätzlichem Phosgen wurde das Reaktionsgemisch im Verlauf von 2 Stunden zum Rückfluss erhitzt. Nach weiteren 2 Stunden Phosgenieren am Rückfluss war eine klare Lösung entstanden. Das fertige Reaktionsgemisch wurde destillativ vom überschüssigen Phosgen und vom Lösungsmittel befreit. Das zurückbleibende Rohprodukt wurde durch Destillation im Vakuum gereinigt. Dabei destillierte 1,2-Bis-(4-isocyanato-3-methyl--phenoxy)-ethan bei 0,5 mbar und 206 bis 218°C ohne Vorlauf als fast farblose Flüssigkeit über, die rasch zu Kristallen vom Schmelzpunkt 111 bis 113°C erstarrte.

Ausbeute: 464 g (96,8% der Theorie)
NCO-Gehalt: ber.: 25,9% gef.: 25,7%
25,9%
Destillationsrückstand: 19 g dunkel gefärbtes, glasartig erstarrendes Harz

Das Diisocyanat liess sich praktisch rückstandsfrei redestillieren und ergab farblose Kristalle vom Schmelzpunkt 113 bis 114°C mit einem Gehalt von hydrolysierbarem Chlor von 20 ppm.

*Beispiel 6*

[Herstellung von 1,4-Bis-(4-isocyanato-3--methyl-phenoxy)-butan]

Es wurden 450 g 1,4-Bis-(4-amino-3-methyl--phenoxy)-butan (analytisch bestimmte Reinheit: 98,2%) entsprechend Beispiel 5b) mit Phosgen umgesetzt. Das dabei gewonnene Rohprodukt wurde durch Vakuumdestillation gereinigt. 1,4-Bis-(4-isocyanato-3-methyl-phenoxy)-butan destillierte ohne Vorlauf bei 0,2 mbar und 205 bis 210°C als farblose Flüssigkeit über, die zu Kristallen vom Schmelzpunkt 89 bis 91°C erstarrte.

Ausbeute: 503 g (97,0% der Theorie)
$C_{20}H_{20}N_2O_4$ (352,4)
NCO-Gehalt: ber.: 23,8% gef.: 23,9%
23,8%
Destillationsrückstand: 21 g dunkles Festprodukt

Das Diisocyanat liess sich praktisch rückstandsfrei redestillieren und ergab farblose Kristalle vom Schmelzpunkt 90 bis 91°C mit einem Gehalt von hydrolysierbarem Chlor von 32 ppm.

**Patentansprüche**

1. Gegebenenfalls in 3-Stellung Methyl-substituierte Bis-(4-isocyanatophenoxy)-alkane der allgemeinen Formel

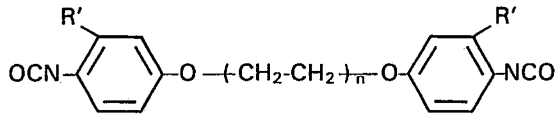

in der R' für Wasserstoff oder eine Methylgruppe steht, wobei beide Reste R' jeweils die gleiche Bedeutung haben, und n für 1, 2 oder 3 steht.

2. 1,2-Bis-(4-isocyanatophenoxy)-ethan.

3. 1,2-Bis-(4-isocyanato-3-methyl-phenoxy)--ethan.

4. Verfahren zur Herstellung von, gegebenenfalls in 3-Stellung Methyl-substituierten, Bis-(4-isocyanatophenoxy)-alkanen der in Anspruch 1 genannten Formel, dadurch gekennzeichnet, dass man gegebenenfalls in 3-Stellung Methyl-substituierte Bis-(aminophenoxy)-alkane der allgemeinen Formel

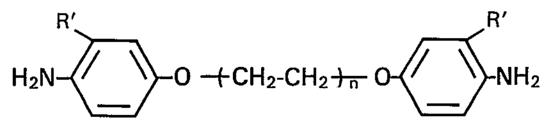

in der R' und n die in Anspruch 1 genannte Bedeutung haben, oder deren Addukte mit Chlorwasserstoff oder Kohlendioxid in an sich bekannter Weise mit Phosgen umsetzt.

5. Verwendung von gegebenenfalls in 3-Stellung Methyl-substituierten Bis-(4-isocyanatophenoxy)--alkanen gemäss Anspruch 1 zur Herstellung von Polyurethankunststoffen nach dem Isocyanat-Polyadditionsverfahren.

**Claims**

1. Bis-(4-isocyanatophenoxy)-alkanes optionally methyl substituted in the 3 position, corresponding to the following general formula:

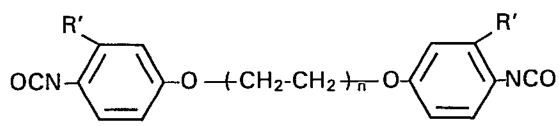

wherein R' denotes hydrogen or a methyl group, the two groups R' being identical, and n stands for 1, 2 or 3.

2. 1,2-bis-(4-isocyanatophenoxy)-ethane.

3. 1,2-bis-(4-isocyanato-3-methyl-phenoxy)-ethane.

4. Process for the preparation of bis-(4-isocyanatophenoxy)-alkanes optionally methyl substituted in the 3 position, corresponding to the formula indicated in Claim 1, characterised in that bis-(aminophenoxy)-alkanes optionally methyl substituted in

the 3 position corresponding to the following general formula:

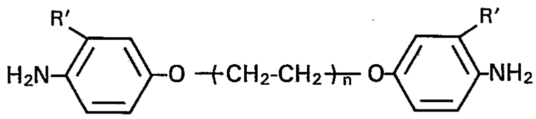

wherein R' and n have the meanings mentioned in Claim 1 or adducts thereof with hydrogen chloride or carbon dioxide are reacted with phosgene in known manner.

5. Use of bis-(4-isocyanatophenoxy)-alkanes optionally substituted in the 3 position according to Claim 1 for the preparation of polyurethanes by the isocyanate polyaddition process.

**Revendications**

1. Bis-(4-isocyanatophénoxy)-alcanes, éventuellement substitués en position 3 par un groupe méthyle, de formule générale:

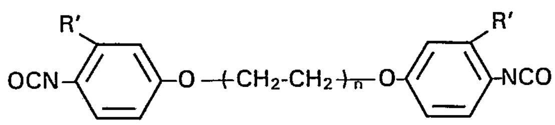

dans laquelle R' représente un atome d'hydrogène ou un groupe méthyle, les deux restes R' ayant à chaque fois la même signification, et n vaut 1, 2 ou 3.

2. Le 1,2-bis-(4-isocyanatophénoxy)-éthane.

3. Le 1,2-bis-(4-isocyanato-3-méthylphénoxy)--éthane.

4. Procédé pour préparer les bis-(4-isocyanatophénoxy)-alcanes, éventuellement substitués en position 3 par un groupe méthyle, ayant la formule indiquée à la revendication 1, procédé caractérisé en ce qu'on fait réagir de façon connue avec le phosgène des bis-(aminophénoxy)-alcanes, éventuellement substitués en position 3 par un groupe méthyle, de formule générale:

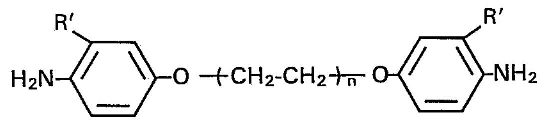

dans laquelle R' et n ont le sens indiqué à la revendication 1, ou leurs produits d'addition avec le chlorure d'hydrogène ou avec l'anhydride carbonique.

5. Utilisation de bis-(4-isocyanatophénoxy)-alcanes, éventuellement substitués en position 3 par un groupe méthyle, selon la revendication 1, pour préparer des matières plastiques en polyurethanne, selon le procédé de polyaddition d'isocyanates.